# EUROPEAN PATENT APPLICATION

(11) **EP 4 339 203 A1**
(43) Date of publication of application: **20.03.2024**
(21) Application number: 22806815.1
(22) Date of filing: 11.05.2022
(51) Int. Cl.: C07K 7/08, A61K 38/10, A61P 35/00

(54) **BECLIN1-TARGETED STAPLED PEPTIDE AND PHARMACEUTICAL COMPOSITION**

(30) Priority: 12.05.2021 CN 202110518586
(71) Applicant: Shenzhen Research Institute Of The Hong Kong Polytechnic University, Shenzhen, Guangdong 518057 (CN); Fudan University, Shanghai 200433 (CN)
(72) Inventor: ZHAO, Yanxiang, Hong Kong Polytechnic University, No.18, Yuexing First Road, South Zone of High-tech Industrial Park, Yuehai Street, Nanshan District Shenzhen, Guangdong 518057 (CN); QIU, Xianxiu, Hong Kong Polytechnic University, No.18, Yuexing First Road, South Zone of High-tech Industrial Park, Yuehai Street, Nanshan District Shenzhen, Guangdong 518057 (CN); ZHANG, Xiaozhe, Hong Kong Polytechnic University, No.18, Yuexing First Road, South Zone of High-tech Industrial Park, Yuehai Street, Nanshan District Shenzhen, Guangdong 518057 (CN); LI, Na, Hong Kong Polytechnic University, No.18, Yuexing First Road, South Zone of High-tech Industrial Park, Yuehai Street, Nanshan District Shenzhen, Guangdong 518057 (CN); WANG, Renxiao, Hong Kong Polytechnic University, No.18, Yuexing First Road, South Zone of High-tech Industrial Park, Yuehai Street, Nanshan District Shenzhen, Guangdong 518057 (CN)
(74) Representative: KIPA AB
(86) International application number: PCT/CN2022/092326
(87) International publication number: WO 2022/237853

(57) **Abstract**

Disclosed are a Beclin 1-targeting stapled peptide and a pharmaceutical composition. The stapled peptide comprises an amino acid sequence at least 66.7% identical to amino acid residues 191-205 of Beclin 1, an amino acid residue E195 and an amino acid residue N202 in the stapled peptide are connected by a hydrocarbon staple to stabilize the α-helical structure of the stapled peptide. The hydrocarbon staple is located at a position in the stapled peptide closer to the Beclin1 coiled coil domain-stapled peptide binding interface, which can enhance the binding affinity of the stapled peptide to Beclin 1 by 10-30 fold. Compared to other autophagy inducers like rapamycin and Tat-Beclin 1 peptide, the optimized stapled peptide of the present disclosure shows the unique profile of not only inducing autophagy but also significantly enhancing the endolysosomal degradation of cell surface oncogenic receptors EGFR and HER2 in HER2-positive cancer cells.

## Description

### FIELD OF THE DISCLOSURE

The present disclosure relates to the technical field of functional polypeptide analogues, in particular to a Beclin 1-targeting stapled peptide and a pharmaceutical composition.

### BACKGROUND

The phosphoinositol-3-kinase (PI3K) complex is a complex in the process of autophagy regulation, and the core components include scaffolding protein.

Beclin 1 and lipid kinase Vps34 (Vacuolar Protein Sorting 34). Through the efficient and specific interaction with Beclin 1, UVRAG forms a complex with Beclin 1-Vps34 to enhance the activity of lipid kinase, thereby promoting cell physiological process related to Vps34, such as autophagy (Liang et al., 2006; Liang et al., 2007). It is also found that UVRAG can cooperate with the class C Vps complexes to regulate endolysosomal trafficking (Liang et al., 2008a; Liang et al., 2008b). In addition, UVRAG can help maintain the structural integrity of chromosomes and the proper chromosome segregation through the interaction with centrosomal protein CEP63 and DNA-PK involved in homologous end joining (Zhao et al., 2012).

Beclin 1 is a scaffolding member of the Class III Phosphoinositol-3-kinase (PI3KC3) complex and is essential for Pt3KC3-mediated cellular processes including autophagy, endolysosomal trafficking and phagocytosis. Beclin 1 is also a haplo-insufficient tumor suppressor and frequently deleted in human sporadic breast, ovarian and prostate cancer. Beclin 1 recruits two PI3KC3 regulators Atg14L and UVRAG through its coiled coil domain to form Atg14L/UVRAG-containing PI3KC3 complex with up-regulated kinase activity. A previous work has shown that the Beclin 1 coiled coil domain forms a metastable homodimer that readily dissociates upon binding ofAtg14L or UVRAG to form a stabilized Beclin 1-Atg14L/UVRAG heterodimer. Hydrocarbon-stapled peptides that bound to Beclin 1 coiled coil domain with moderate affinity are developed, to reduce Beclin 1 homodimerization and promote Beclin 1-Atg14L/UVRAG interaction. These peptides also induced autophagy and enhanced endolysosomal degradation of cell surface receptors like EGFR.

However, the previously developed stapled peptide has insufficient affinity to be bound to the Beclin 1 coiled coil domain, and the ability to induce autophagy and enhance the lysosomal degradation of cell surface receptors such as EGFR is not strong enough.

Therefore, the prior art still needs to be improved and developed.

### BRIEF SUMMARY OF THE DISCLOSURE

In view of the above deficiencies in the prior art, the object of the present disclosure is to provide a Beclin 1-targeting stapled peptide and a pharmaceutical composition.

The technical scheme is as follows:
A Beclin 1-targeting stapled peptide, which comprises an amino acid sequence that is at least 66.7% identical to amino acid residues 191-205 of Beclin 1, and an amino acid residue E195 and an amino acid residue N202 in the stapled peptide are connected by a hydrocarbon staple to stabilize an α-helical structure of the stapled peptide.

The Beclin 1-targeting stapled peptide comprises the amino acid sequence that adopts the amino acid residues 191-205 of Beclin 1 as a template, and has 1-5 mutations of the amino acid compared with the amino acid residues 191-205 of Beclin 1.

In one embodiment, the Beclin 1-targeting stapled peptide has the amino acid sequence of one of SEQ ID NO.1-75.

The Beclin 1-targeting stapled peptide, includes one (R)-2-(7-octenyl) alanine residue (R8 residue) and one (S)-2-(4-pentenyl) alanine residue (S5 residue).

A pharmaceutical composition, which includes the stapled peptide disclosed in the present disclosure.

A method for promoting autophagy or endosomal-lysosomal trafficking, which includes steps of treating cells with the stapled peptide disclosed in the present disclosure to enhance lysosomal degradation of one or more target proteins.

In the method, the target protein is an epidermal growth factor receptor.

In the method, a cell proliferation driven by the epidermal growth factor receptor is reduced after the cells are treated with the stapled peptide.

A method for inhibiting the growth of cancer cells, which includes steps of administering the stapled peptide disclosed in the present disclosure to a subject in need.

In the method, the growth of the cancer cells includes a cell proliferation driven by the epidermal growth factor receptor.

Beneficial effects: Compared with the prior art, the present disclosure optimizes the Beclin 1-targeting stapled peptide by adjusting the position of installing the hydrocarbon staple and the amino acid sequence of the stapled peptide. The result shows that placing the hydrocarbon staple closer to the Beclin 1-peptide binding interface enhanced the binding affinity by ~10-30 fold. Compared to other autophagy inducers like rapamycin and Tat-Beclin 1 peptide, the optimized peptides in the present disclosure show the unique profile of not only inducing autophagy but also significantly enhancing the endolysosomal degradation of cell surface oncogenic receptors EGFR and HER2 in HER2-positive cancer cells. Given the importance of EGFR and HER2 signaling pathways in cancer cell proliferation, the Beclin 1-targeting stapled peptides in the present disclosure may serve as effective therapeutic candidates for EGFR- or HER2-driven cancer.

### BRIEF DESCRIPTION OF THE DRAWINGS

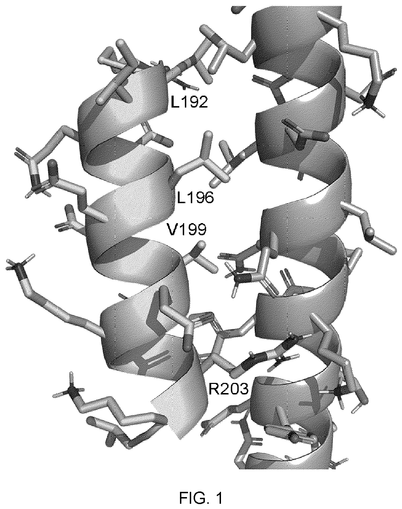

Fig. 1 is a schematic diagram showing the molecular model of the native-P1 peptide (in yellow) in complex with the Beclin-1 coiled coil domain in the present disclosure. The complex model is used as the template for deriving the initial model of all designed stapled peptides described in the present disclosure.

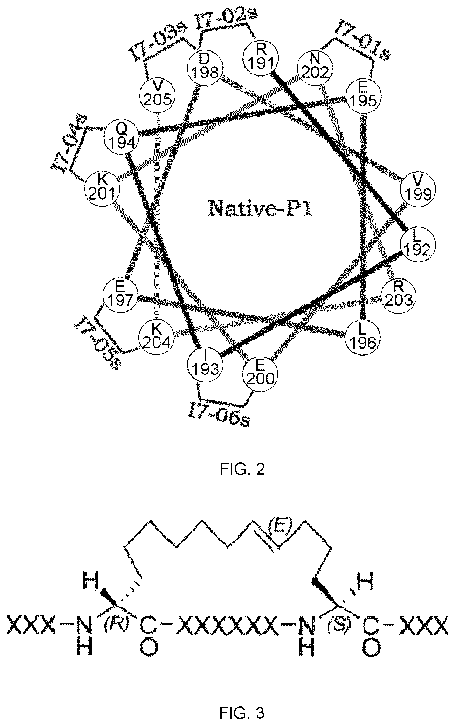

Fig. 2 is a schematic diagram showing the staple position scanning in peptide design, the hydrocarbon staple is added at six positions on the Native-P1 sequence.
Fig. 3 is a schematic diagram showing the chemical structure of the hydrocarbon staple. Two amino acids containing olefin side chains are separated by six residues on the peptide sequence. The two side chains are connected by a Ruthenium-catalyzed ring-closing metathesis reaction, resulting in the 11-carbon long linker shown in this figure.

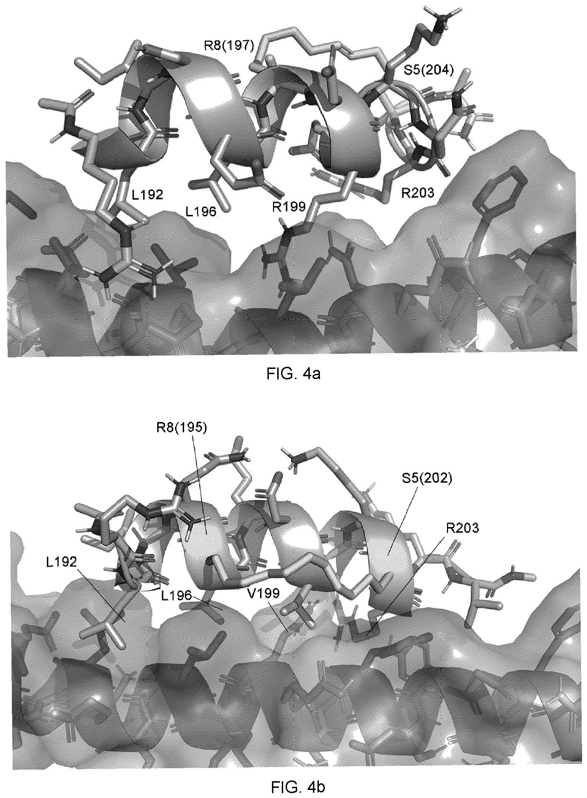

Fig. 4a is a schematic diagram showing the binding mode to Beclin 1 coiled coil domain of the Tat-SP4 obtained in a previous study, Fig. 4b is a schematic diagram showing the binding mode to Beclin 1 coiled coil domain of the newly designed I7-01s scaffold.

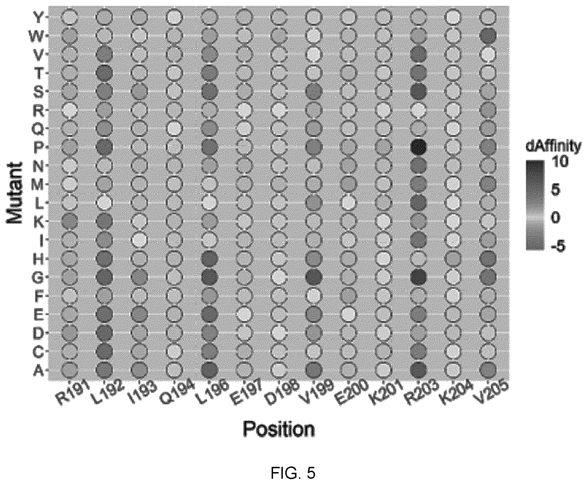

Fig. 5 is a result diagram showing the predicted binding energies of single point mutants based on the i7-01s scaffold. "dAffinity" refers to the difference between the predicted binding energy of a certain mutant and the i7-01s. The unit of binding energy is kcal per mole, and the more negative the value, the higher the binding energy.

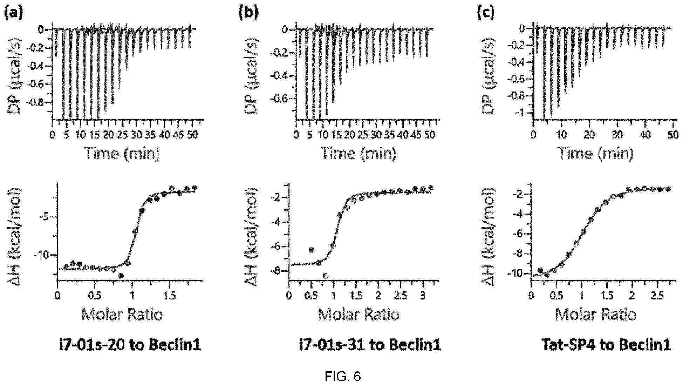

Fig. 6 is a comparative diagram of the binding affinity of I7-01s-20 (a), I7-01s-31 (b) and Tat-SP4 to the Beclin 1 coiled coil domain. Representative ITC profiles for I7-01s-20 (a) and I7-01s-31 (b) show significantly enhanced binding affinity to Beclin1 as compared to Tat-SP4(c).

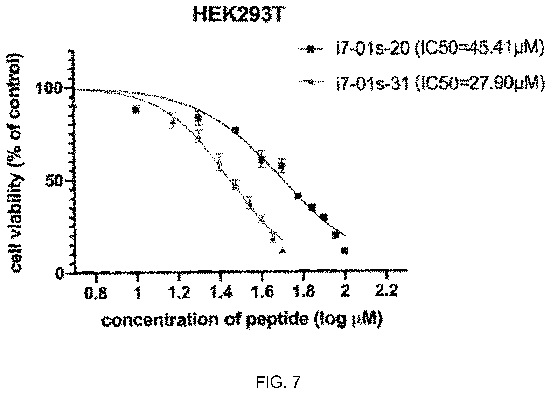

Fig. 7 is a schematic diagram of the result of evaluating the cytotoxicity of the newly designed stapled peptide on HEK293T cells by the trypan blue exclusion assay. For HEK293T cells, treatment with 10 µM i7-01s-20 and 5 µM i7-01s-31 for 24 hours show little impact on cell viability.

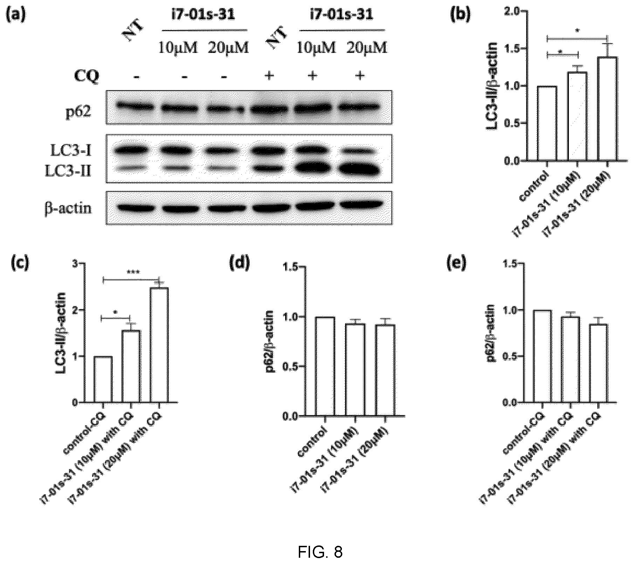

Fig. 8 is a schematic diagram showing that the peptides based on the i7-01s scaffold are effective inducers of autophagy. (a) Western blot to assess the p62 level and LC3 lipidation profile in HEK293T cells after treatment with 10- and 20- µM) i7-01s-31 for 3 hours, in the presence or absence of CQ. (b)&(c) Quantification of LC3 lipidation profiles from the Western blot data. (d)&(e) Quantification of p62 levels from the Western blot data from (a).

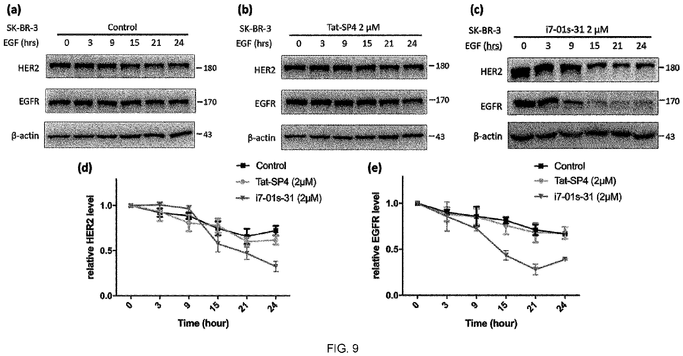

Fig. 9 is a schematic diagram of the result showing peptides based on (I7-01s) scaffold are potent inducers of endolysosomal trafficking. (a-c) Western blot to assess the HER2 and EGFR levels in SKBR3 cells. Cells are starved overnight and are treated with 200 ng/mL EGF respectively with vehicle control (a), 2µM Tat-SP4 (b), 2µM i7-01s-31 (c). (d & e) Time-dependent plots to quantify HER2 and EGFR degradation profiles after three independent experiments. The levels of HER2 (d) and EGFR (e) are normalized to the β-actin level at different time points and then further normalized to time 0, when the ligand EGF is added. Data are presented as mean ± SEM (n = 3).

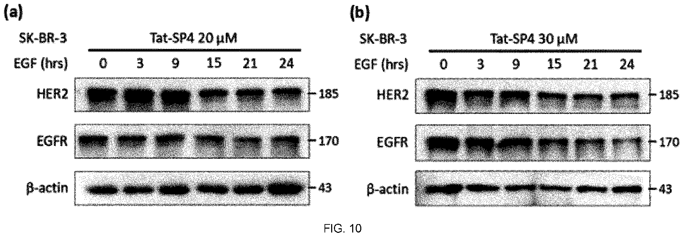

Fig. 10 is a result diagram showing the effect of Tat-SP4 on the degradation of HER2 and EGFR in SKBR3 cells evaluating with Western blot. Compared with i7-01s-31, Tat-SP4 is a moderate regulator on promoting the degradation of HER2 and EGFR.

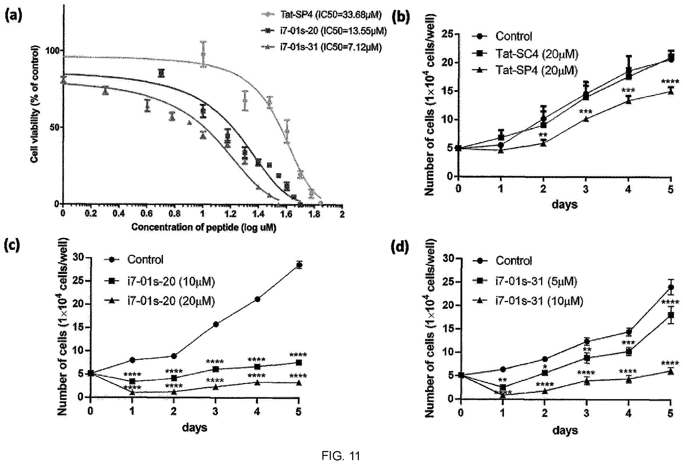

Fig. 11 is a result diagram showing the peptides based on (I7-01s) scaffold exert potent anti-proliferative effect on HER2+ breast cancer cell line. (a) Trypan blue exclusion assay to assess the cytotoxicity IC₅₀ (half maximal inhibitory concentration) of the indicated peptides in SKBR3 cells. Cells are treated with various concentrations of the indicated peptides for24h. Cell numbers are manually counted by the trypan blue exclusion assay using a hemocytometer. Data are presented as mean ± SD from three independent experiments. (b-d) Cell proliferation assay is performed over a time span of five days in SKBR3 cells after treatment with the indicated peptides. Cells are treated with the indicated peptides at day 0 and the cell number is calculated at the given time points. The assay is performed in 96 well plates with three independent measurements. Tat-SC4 is a control peptide with scrambled Tat-SP4 sequence but without the hydrocarbon staple.

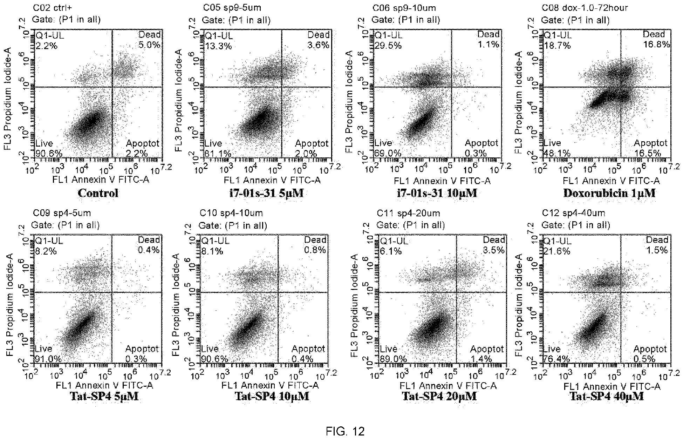

Fig. 12 is a schematic diagram of the result showing the peptides based on strategy (i7-01s) scaffold induce cell death with necrotic features. Flow cytometry analysis with Annexin V FITC-PI staining is performed in SKBR3 cells after treatment of the indicated peptides for 24 hours. The percentage of dead cells (PI positive) in i7-01s-31 treatment groups are significantly increased compared with that of control, without notable changes in the population of apoptotic cells.

### DETAILED DESCRIPTION OF EMBODIMENTS

The present disclosure provides a Beclin 1-targeting stapled peptide. In order to make the purpose, technical solution and effect of the present disclosure clearer and more definite, the present disclosure is further described in detail below. It should be understood that the embodiments described here are only used to explain the present disclosure, not to limit the present disclosure.

Those skilled in the art should understand that unless otherwise stated, the singular forms "a", "an", "said" and "the" used herein may also include plural forms. It should be further understood that the word "comprising" used in the description of the present disclosure refers to the presence of said features, integers, steps, operations, elements and/or components, but does not exclude the presence or addition of one or more other features, Integers, steps, operations, elements, components, and/or groups thereof. It should be understood that when an element is referred to as being "connected" or "coupled" to another element, it can be directly connected or coupled to the other element or intervening elements may also be present. Additionally, "connected" or "coupled" as used herein may include wireless connection or wireless coupling. The expression "and/or" used herein includes all of one or more associated listed items or combinations of any element and the all.

Those skilled in the art can understand that, unless otherwise defined, all terms (including technical terms and scientific terms) used herein have the same meaning as commonly understood by those of ordinary skill in the art to which the present disclosure belongs. It should also be understood that the terms, such as those defined in commonly used dictionaries, should be interpreted as having meanings consistent with those meanings in the context of the prior art, and unless specifically defined as herein, the terms are not intended to be explained by idealized or over formal meanings.

Beclin 1 (Bcl-2 interacting coiled coil protein 1) is an evolutionarily conserved mammalian protein with homologs identified in eukaryotes ranging from yeast and plants to C. elegans, fruit flies and zebrafish. Initially discovered as a Bcl-2 interacting protein in a yeast two-hybrid screen, Beclin 1 has later been confirmed to be an indispensable member of the mammalian Class III phosphatidylinositol 3-kinase (PI3KC3) complex. PI3KC3 is a multi-protein assembly with a core unit formed by three molecules including Beclin 1, the lipid kinase Vps34 (Vacuolar Protein Sorting 34) that specifically phosphorylates phosphatidylinositols (Pls) at the 3' hydroxyl position of the inositol ring to generate PI3Ps; and a serine/threonine kinase Vps15 (Vacuolar Protein Sorting 15) that constitutively associates with Vps34 as a stable binding partner. PI3KC3 serves as the major producer of phosphatidylinositol-3-phosphates (Pl3Ps) in mammalian cells and is essential for cellular processes that involve PI3P-enriched membrane vesicles including autophagy, endolysosomal trafficking and phagocytosis. As a critical regulator of the PI3KC3 complex, Beclin 1 has been functionally implicated in a variety of physiological processes such as glucose metabolism, embryonic development and innate immunity. Deficiency or dysfunction of Beclin 1 has been linked to human pathologies such as cancer and neurodegenerative diseases.

Extensive studies have confirmed Beclin 1 as a haplo-insufficient tumor suppressor intimately involved in tumorigenesis. Beclin 1 is monoallelically deleted in 40-75% of cases of human sporadic breast, ovarian, and prostate cancer. Heterozygous knockout of Beclin1 in mice leads to higher rate of spontaneous malignancies such as lymphomas and lung or hepatocellular carcinomas. Additionally, cell surface oncogenic receptors such as Epidermal Growth Factor Receptor (EGFR) in non-small-cell lung cancer (NSCLC) and Human Epidermal Growth Factor Receptor 2 (HER2) in breast cancer can associate with Beclin 1 to suppress autophagy and promote tumorigenesis. Furthermore, induction of autophagy by over-expressing a Beclin 1 F121A mutant that doesn't bind to its inhibitor Bcl-2 has been shown to enhance autophagy and abrogate tumorigenesis in transgenic mice that over-express HER2. Notably, Tat-Beclin 1, a peptide that contains the HIV Tat sequence for cell penetration and a segment of 11 amino acids from the evolutionarily conserved region of Beclin 1 (residue 269 to 279) shows potent anti-proliferative effect in mice xenograft model of HER2-positive (HER2+) human breast tumor, with efficacy comparable to the clinically used HER2 inhibitor lapatinib. These genetic and pharmacological findings suggest that targeting Beclin 1 may serve as an effective anti-proliferative strategy for EGFR- or HER2-driven cancer.

Structural studies have delineated the molecular mechanism of how Beclin 1 promotes the activity of the PI3KC3 complex. As the scaffolding molecule, Beclin 1 directly recruits two positive regulators Atg14L and UVRAG in mutually exclusive manner to form Atg14L- or UVRAG-containing PI3KC3 complexes with significantly enhanced lipid kinase activity to promote PI3KC3-dependent processes. The Beclin 1-Atg14L/UVRAG interaction relies critically on their respective coiled coil domains. In particular, the present studies reveal that the Beclin 1 coiled coil domain forms a metastable homodimer because its hydrophobic dimer interface is significantly weakened by several "imperfect" polar or charged residues. The metastable Beclin 1 homodimer readily dissociates upon Atg14L/UVRAG binding and transits into highly stable heterodimeric Beclin 1-Atg14L/UVRAG complex with a coiled coil interface significantly strengthened by hydrophobic pairings and electrostatically complementary interactions. Harnessing the metastable feature of Beclin 1 coiled coil domain, a series of hydrocarbon-stapled peptides that specifically bind to this region are developed to reduce Beclin 1 homodimerization and enhance the Beclin 1-Atg14L/UVRAG interaction. These designed peptides also showed efficacy in terms of inducing autophagy and promoting the endolysosomal degradation of cell surface receptors like EGFR.

Given the functional importance of Beclin 1 in tumorigenesis, the rationally designed Beclin 1-targeting stapled peptides may inhibit the proliferation of EGFR- or HER2-driven cancer cells by enhancing autophagy and promoting the endolysosomal degradation of EGFR and HER2. However, the peptides developed in the previous study have moderate in vitro binding affinity to Beclin 1 (Kd ~ 6-60 µM) and show little cytotoxicity in multiple NSCLC cell lines.

Based on this, the present disclosure provides a novel Beclin 1-targeting stapled peptide, which comprises an amino acid sequence that is at least 66.7% identical to the amino acid residues 191-205 of Beclin 1, the amino acid residue E195 and the amino acid residue N202 in the stapled peptide are connected by a hydrocarbon staple to stabilize the α-helical structure of the stapled peptide.

The stapled peptide selected in the present disclosure is prepared by adopting amino acid residues 191-205 (Native-P1 peptide) on the Beclin 1 coiled coil domain as a template, and performing amino acid mutation and modification on the template. The present disclosure chooses Native-P1 peptide as the template is because studies have shown that the fragment forms part of the Beclin 1 homodimer interface but does not overlap with the binding site for UVRAG. Thus, amino acid residues 191-205 on the Beclin 1 coiled coil domain (Native-P1 peptide) are expected to bind to the Beclin 1 coiled coil domain to reduce the homodimerization and promote the Beclin 1-Atg14I/UVRAG heterodimeric complex, subsequently leading to enhanced autophagy and endolysosomal trafficking.

When the Native-P1 peptide binds to the Beclin 1 coiled coil domain, an α-helix structure as shown in Fig. 1 is formed. In the present disclosure, a hydrocarbon staple is adopted to link two amino acid residues (namely amino acid residue E195 and amino acid residue N202) that are separated by 6 residues on the Native-P1 sequence, so that the staple can cross two rounds of helical circle to stabilize the peptide structure.

As shown in Fig. 2, the present disclosure attempts six possible scaffolds for installing this hydrocarbon staple, all of which keep the four residues critical for binding (i.e. L192, L196, V199, and R203) undisturbed. During MD simulations, the helical structures of all six stapled peptides are maintained especially between residues L192 to R203. Although the MD simulations, 100 ns long for each, are not extensive enough to reach real conformational convergence, the predicted binding energies of all six stapled peptides indicate that i7-01s, i7-02s, i7-04s, and i7-06s tend to form more stable interactions with the Beclin 1 coiled coil domain.

As shown in Fig. 3, the present disclosure chooses the i7-01s scaffold for further analysis because in this case, amino acid residue E195 and amino acid residue N202 on Native-P1 are mutated to install the hydrocarbon staple, both of which are near the hydrophobic region on the surface of the coiled coil domain. Thus, as shown in Fig. 4, the mutation of amino acid residue E195 and amino acid residue N202 may reduce the unfavorable hydrophobic-hydrophilic mismatch between the peptide and the coiled coil domain. Besides, according to the predicted model, the hydrocarbon staple on i7-01s actually stacks on the surface of the coiled coil domain, which may further enhance the binding of this stapled peptide. Based on the i7-01s scaffold, systematic single-point mutations are conducted on the Native-P1 sequence. As shown in Fig. 5, the predicted binding energies of all of those mutated peptides indicate that mutations at Q194, E197, D198, E200, K201, K204, and K205 are more likely to improve binding affinity. Then certain favorable single-point mutants are manually selected and combined into new designs of multiple-point mutants, in a hope to achieve even higher binding affinity. In particular, the stapled peptides comprise the amino acid sequences that adopt Beclin 1 amino acid residues 191-205 as template, and have 1-5 amino acid mutations comparing to the Beclin 1 amino acid residues 191-205. Binding energies for all 75 peptides designed are summarized in Table 1. The average binding energy of most of them (72 in 75) is more favorable than that of the Native-P1 peptide based on the same i7-01s scaffold, which meet the expectation on molecular design.

**Table 1 Binding energy of designed peptides based on i7-01s scaffold**

| ID | SEQUENCE^{a} | MM-GB/SA Binding Energy (kcal/mol)^{b} | | |
|---|---|---|---|---|
| | | Job1 | Job2 | Job3 |
| i7-01s | Ace-RLIQR8LEDVEKS5RKV-Nme | -34.56 ± 4.85 | -27.48 ± 4.76 | -37.47 ± 4.43 |
| i7-01s-01 | Ace-RLIQR8LLDREAS5RIV-Nme | -42.24 ± 6.72 | -38.17 ± 5.27 | -50.88 ± 5.98 |
| i7-01s-02 | Ace-RLIQR8REDREKS5RAV-Nme | -69.59 ± 6.04 | -48.27 ± 8.12 | -47.31 ± 4.19 |
| i7-01s-03 | Ace-RLIQR8TEDSEKS5RAV-Nme | -32.19 ± 4.66 | -38.16 ± 4.67 | -35.72 ± 5.35 |
| i7-01s-04 | Ace-RLISR8LEDRDKS5RKV-Nme | -31.77 ± 4.72 | -46.10 ± 9.75 | -37.82 ± 4.82 |
| i7-01s-05 | Ace-RLIQR8REDREKS5RKV-Nme | -39.76 ± 8.07 | -48.30 ± 6.27 | -46.32 ± 5.00 |
| i7-01s-06 | Ace-RLISR8LLDREKS5RGV-Nme | -42.31 ± 6.28 | -37.73 ± 3.89 | -57.78 ± 5.32 |
| i7-01s-07 | Ace-RLISR8LLDREAS5RAV-Nme | -38.40 ± 7.63 | -47.44 ± 6.75 | -27.35 ± 6.33 |
| i7-01s-08 | Ace-LKIQR8KEDVEKS5RKV-Nme | -34.61 ± 8.95 | -38.23 ± 5.65 | -40.26 ± 4.39 |
| i7-01s-09 | Ace-LLIQR8KEDVEKS5RAV-Nme | -38.71 ± 4.32 | -38.80 ± 4.99 | -39.54 ± 4.62 |
| i7-01s-10 | Ace-RLIQR8REDREKS5RAR-Nme | -59.91 ± 4.41 | -65.15 ± 5.37 | -72.09 ± 8.24 |
| i7-01s-11 | Ace-RLIQR8REDREKS5RAE-Nme | -52.99 ± 10.08 | -33.80 ± 7.56 | -41.61 ± 5.23 |
| i7-01s-12 | Ace-RLISR8REDREKS5RAE-Nme | -43.37 ± 4.35 | -44.90 ± 4.54 | -25.97 ± 7.27 |
| i7-01s-13 | Ace-RLIQR8REDVEKS5RAE-Nme | -37.00 ± 6.75 | -45.77 ± 8.74 | -41.99 ± 4.74 |
| i7-01s-14 | Ace-LLIQR8RLDREAS5RAE-Nme | -27.11 ± 7.42 | -38.85 ± 4.25 | -21.51 ± 6.34 |
| i7-01s-15 | Ace-RLISR8REDREKS5RAV-Nme | -33.13 ± 8.26 | -34.14 ± 8.43 | -35.09 ± 5.70 |
| i7-01s-16 | Ace-YLIFR8LTDLEKS5RVV-Nme | -41.75 ± 4.02 | -29.17 ± 4.37 | -33.22 ± 7.47 |
| i7-01s-17 | Ace-CLIWR8LIDVLKS5RAV-Nme | -35.14 ± 4.47 | -37.62 ± 3.91 | -36.67 ± 4.02 |
| i7-01s-18 | Ace-MLIDR8LIDWKS5RW-Nme | -38.26 ± 3.65 | -38.53 ± 3.95 | -31.71 ± 5.74 |
| i7-01s-19 | Ace-RLIIR8LRWKERS5RKV-Nme | -38.14 ± 10.25 | -28.48 ± 6.67 | -34.53 ± 5.31 |
| i7-01s-20 | Ace-RVIQR8LVIIEKS5RDV-Nme | -28.34 ± 4.67 | -27.08 ± 4.68 | -41.60 ± 7.98 |
| i7-01s-21 | Ace-RLIVR8LHDRIKS5RLV-Nme | -39.30 ± 5.35 | -40.19 ± 4.12 | -30.85 ± 6.79 |
| i7-01s-22 | Ace-RLIKR8LFYVEKS5RCI-Nme | -41.90 ± 5.69 | -35.72 ± 5.36 | -39.50 ± 5.36 |
| i7-01s-23 | Ace-RLIQR8LTIVLIS5RRV-Nme | -38.89 ± 3.73 | -38.96 ± 4.74 | -34.56 ± 4.55 |
| i7-01s-24 | Ace-YLIQR8LIYVEKS5RQI-Nme | -38.06 ± 4.36 | -34.29 ± 4.60 | -35.69 ± 4.04 |
| i7-01s-25 | Ace-RLPER8IMDVIKS5RKV-Nme | -40.01 ± 4.16 | -39.35 ± 3.98 | -33.01 ± 6.01 |
| i7-01s-26 | Ace-QLIQR8LFYVLKS5IKV-Nme | -36.23 ± 2.88 | -34.75 ± 2.99 | -38.31 ± 6.20 |
| i7-01s-27 | Ace-WLFQR8LLDVEKS5IVV-Nme | -34.86 ± 3.87 | -34.61 ± 4.12 | -35.01 ± 3.98 |
| i7-01s-28 | Ace-RLIMR8KEDVLKS5RSL-Nme | -38.52 ± 5.02 | -37.52 ± 6.47 | -38.88 ± 4.74 |
| i7-01s-29 | Ace-RLIRR8VEDVLKS5KLV-Nme | -33.93 ± 4.44 | -33.57 ± 3.78 | -34.84 ± 3.99 |
| i7-01s-30 | Ace-RLLQR8LKTVLKS5RSV-Nme | -39.06 ± 5.76 | -40.13 ± 4.10 | -40.32 ± 4.04 |
| i7-01s-31 | Ace-VLFNR8LVDVIKS5RKV-Nme | -32.19 ± 4.21 | -40.66 ± 3.58 | -36.60 ± 4.73 |
| i7-01s-32 | Ace-ELISR8LFFVEKS5RTV-Nme | -34.48 ± 4.59 | -38.88 ± 4.58 | -37.69 ± 4.24 |
| i7-01s-33 | Ace-RLIQR8LVRVGKS5ISV-Nme | -36.45 ± 5.39 | -36.21 ± 4.43 | -34.37 ± 3.98 |
| i7-01s-34 | Ace-RFIQR8LEVVIKS5RSV-Nme | -45.61 ± 8.26 | -45.12 ± 5.95 | -51.54 ± 5.95 |
| i7-01s-35 | Ace-DLIFR8LWYVEKS5IKV-Nme | -45.61 ± 8.27 | -45.12 ± 5.96 | -51.54 ± 5.96 |
| i7-01s-36 | Ace-RLIQR8REDVEKS5RKR-Nme | -55.68 ± 5.61 | -55.78 ± 6.04 | -58.27 ± 5.82 |
| i7-01s-37 | Ace-RLIQR8KEDVEKS5RKR-Nme | -53.42 ± 5.02 | -50.71 ± 4.70 | -51.34 ± 4.81 |
| i7-01s-38 | Ace-RLIQR8YEDVEKS5RKR-Nme | -53.13 ± 6.06 | -45.87 ± 8.18 | -51.42 ± 5.39 |
| i7-01s-39 | Ace-RLIQR8WWDVEKS5RKV-Nme | -35.57 ± 7.77 | -39.81 ± 6.83 | -41.67 ± 3.77 |
| i7-01s-40 | Ace-RLIQR8EEDVEKS5RKR-Nme | -47.79 ± 4.78 | -48.32 ± 9.42 | -42.12 ± 6.33 |
| i7-01s-41 | Ace-RLIQR8HEDVEKS5RKR-Nme | -51.92 ± 4.34 | -50.94 ± 5.62 | -50.95 ± 5.66 |
| i7-01s-42 | Ace-RLIQR8WFDVEKS5RKV-Nme | -50.02 ± 4.08 | -48.69 ± 7.76 | -43.80 ± 4.44 |
| i7-01s-43 | Ace-RLlQR8WSDVEKS5RKV-Nme | -39.21 ± 4.60 | -45.46 ± 4.11 | -36.47 ± 7.33 |
| i7-01s-44 | Ace-RLIQR8EEDVEKS5RKW-Nme | -30.51 ± 4.30 | -35.21 ± 5.47 | -33.21 ± 4.79 |
| i7-01s-45 | Ace-RLKQR8LEDVEKS5RKR-Nme | -44.58 ± 6.20 | -48.82 ± 5.29 | -49.16 ± 4.98 |
| i7-01s-46 | Ace-RLIQR8KIDVEKS5RKR-Nme | -51.94 ± 4.43 | -52.88 ± 4.93 | -60.71 ± 8.81 |
| i7-01s-47 | Ace-YLIQR8MEDVEKS5RKR-Nme | -49.59 ± 5.98 | -51.88 ± 4.05 | -52.77 ± 6.27 |
| i7-01s-48 | Ace-YLIQR8QEDVEKS5RKR-Nme | -38.40 ± 5.07 | -47.23 ± 5.82 | -49.75 ± 5.11 |
| i7-01s-49 | Ace-WLIQR8WTDVEKS5RKV-Nme | -43.61 ± 5.20 | -43.66 ± 4.57 | -38.97 ± 6.08 |
| i7-01s-50 | Ace-RLIQR8MEDVEES5RKR-Nme | -40.28 ± 4.97 | -40.56 ± 4.28 | -39.32 ± 4.34 |
| i7-01s-51 | Ace-RLIQR8MGDVEKS5RKR-Nme | -52.66 ± 4.69 | -52.66 ± 5.01 | -47.99 ± 5.23 |
| i7-01s-52 | Ace-RLRQR8LEDVEKS5RFW-Nme | -41.51 ± 5.99 | -29.64 ± 3.85 | -28.72 ± 5.06 |
| i7-01s-53 | Ace-YLIQR8WMDVEKS5RKV-Nme | -44.80 ± 3.68 | -46.01 ± 4.36 | -44.97 ± 4.04 |
| i7-01s-54 | Ace-ALIQR8QEDVEKS5RKR-Nme | -59.35 ± 4.57 | -49.49 ± 4.38 | -54.90 ± 5.66 |
| i7-01s-55 | Ace-RLIQR8FEDVLKS5RKR-Nme | -51.16 ± 4.27 | -45.01 ± 6.00 | -52.37 ± 6.21 |
| i7-01s-56 | Ace-RLNQR8WNDVEKS5RKW-Nme | -41.98 ± 5.00 | -34.80 ± 3.83 | -38.36 ± 5.30 |
| i7-01s-57 | Ace-RRIQR8MEDVLKS5RKR-Nme | -58.39 ± 5.91 | -50.86 ± 5.54 | -50.04 ± 5.62 |
| i7-01s-58 | Ace-RLIER8REIVEKS5RKR-Nme | -50.33 ± 5.09 | -50.43 ± 4.98 | -51.21 ± 5.89 |
| i7-01s-59 | Ace-QLIQR8RSDVEKS5RKW-Nme | -37.81 ± 5.27 | -41.05 ± 4.56 | -43.92 ± 4.47 |
| i7-01s-60 | Ace-RLIQR8KCGVEKS5RKW-Nme | -41.35 ± 4.64 | -40.75 ± 5.22 | -38.75 ± 6.06 |
| i7-01s-61 | Ace-RLIQR8MEDVTKS5RIW-Nme | -40.07 ± 3.86 | -40.60 ± 4.74 | -39.84 ± 4.40 |
| i7-01s-62 | Ace-RRINR8KEDVEKS5RKK-Nme | -53.98 ± 5.67 | -42.98 ± 6.04 | -47.36 ± 7.86 |
| i7-01s-63 | Ace-RLIQR8YHDVDKS5RKR-Nme | -55.33 ± 4.96 | -41.00 ± 6.14 | -52.53 ± 6.04 |
| i7-01s-64 | Ace-RLIIR8QEDVEKS5RKR-Nme | -50.48 ± 4.37 | -50.75 ± 6.28 | -47.12 ± 5.49 |
| i7-01s-65 | Ace-RRILR8LMDVEKS5RKR-Nme | -52.61 ± 7.33 | -54.55 ± 6.91 | -43.41 ± 4.57 |
| i7-01s-66 | Ace-RLIQR8WWSVDKS5RKW-Nme | -45.97 ± 4.66 | -37.64 ± 5.04 | -53.77 ± 4.82 |
| i7-01s-67 | Ace-RLIQR8WKSVEKS5RGW-Nme | -35.39 ± 5.50 | -44.35 ± 4.17 | -43.17 ± 4.70 |
| i7-01s-68 | Ace-RLIHR8KEDVETS5RQR-Nme | -60.56 ± 5.05 | -41.96 ± 6.34 | -51.51 ± 8.10 |
| i7-01s-69 | Ace-RLKQR8KSDVEKS5RKW-Nme | -40.57 ± 4.12 | -41.35 ± 7.63 | -35.64 ± 5.96 |
| i7-01s-70 | Ace-RLIQR8KGDVELS5RKR-Nme | -49.73 ± 6.45 | -50.68 ± 5.87 | -55.95 ± 6.13 |
| i7-01s-71 | Ace-RLINR8KEWVEHS5RKW-Nme | -29.22 ± 4.36 | -38.17 ± 5.42 | -44.24 ± 8.73 |
| i7-01s-72 | Ace-RLIQR8RQDVRIS5RKW-Nme | -42.19 ± 4.87 | -43.19 ± 4.95 | -43.01 ± 5.21 |
| i7-01s-73 | Ace-RLKHR8WGSVEKS5RKV-Nme | -28.59 ± 6.63 | -42.50 ± 5.25 | -39.76 ± 4.76 |
| i7-01s-74 | Ace-RLIMR8RETNEKS5RKW-Nme | -48.12 ± 5.33 | -43.90 ± 4.39 | -33.42 ± 5.28 |
| i7-01s-75 | Ace-RLIYR8MEDVEKS5RKR-Nme | -51.08 ± 4.84 | -50.71 ± 4.14 | -49.97 ± 4.29 |

Among all designs, 17 stapled peptides are selected for chemical synthesis based on a balanced consideration of their predicted binding mode and binding affinity as well as their physicochemical properties (such as the total charge). The binding affinities of all 17 newly synthesized stapled peptides to the Beclin 1 coiled coil domain are measured by isothermal titration calorimetry (ITC), as shown in Table 2.

**Table 2 Binding affinities of 17 newly synthesized stapled peptides to the Beclin 1 coiled coil domain**

| ID | SEQUENCE * | K_{d} (µM) ^{#} |
|---|---|---|
| Native-P1 | RLIQELEDVEKNRKV | |
| Tat-SP4 | Ac-[Tat]-RLISEL(R8)DREKQR(S5)A-NH₂ | 3.21 ± 0.49 |
| i7-01s-02 | Ac-[Tat]-RLIQ(R8)REDREK(S5)RAV-NH₂ | 0.86 ± 0.16 |
| i7-01s-10 | Ac-[Tat]-RLIQ(R8)REDREK(S5)RAR-NH₂ | 2.03 ± 0.56 |
| i7-01s-12 | Ac-[Tat]-RLIS(R8)REDREK(S5)RAE-NH₂ | 2.84 ± 0.48 |
| i7-01s-20 | Ac-[Tat]-RVIQ(R8)LVIIEK(S5)RDV-NH₂ | 0.10 ±0.05 |
| i7-01s-30 | Ac-[Tat]-RLLQ(R8)LKTVLK(S5)RSV-NH₂ | No binding |
| i7-01s-31 | Ac-[Tat]-VLFN(R8)LVDVIK(S5)RKV-NH₂ | 0.33 ± 0.28 |
| i7-01s-34 | Ac-[Tat]-RFIQ(R8)LEVVIK(S5)RSV-NH₂ | No binding |
| i7-01s-36 | Ac-[Tat]-RLIQ(R8)REDVEK(S5)RKR-NH₂ | No binding |
| i7-01s-37 | Ac-[Tat]-RLIQ(R8)KEDVEK(S5)RKR-NH₂ | 2.41 ± 0.35 |
| i7-01s-45 | Ac-[Tat]-RLKQ(R8)LEDVEK(S5)RKR-NH₂ | 1.41 ± 0.32 |
| i7-01s-46 | Ac-[Tat]-RLIQ(R8)KIDVEK(S5)RKR-NH₂ | 0.71 ± 0.23 |
| i7-01s-54 | Ac-[Tat]-ALIQ(R8)QEDVEK(S5)RKR-NH₂ | 311 ± 54.62 |
| i7-01s-57 | Ac-[Tat]-RRIQ(R8)MEDVLK(S5)RKR-NH₂ | 2.20 ± 0.36 |
| i7-01s-58 | Ac-[Tat]-RLIE(R8)REIVEK(S5)RKR-NH₂ | 0.61 ± 0.17 |
| i7-01s-64 | Ac-[Tat]-RLII(R8)QEDVEK(S5)RKR-NH₂ | 356 ± 78.53 |
| i7-01s-70 | Ac-[Tat]-RLIQ(R8)KGDVEL(S5)RKR-NH₂ | 2.14 ± 0.47 |
| i7-01s-75 | Ac-[Tat]-RLIY(R8)MEDVEK(S5)RKR-NH₂ | 1.29 ± 0.40 |

Compared to Tat-SP4, which is the best-affinity stapled peptide (Kd = 3.2 µM) obtained in previous study, 12 of the newly synthesized peptides exhibit comparable or stronger binding to Beclin 1. In particular, as shown in Fig. 6, two peptides (i.e. i7-01s-20 and i7-01s-31) have binding affinity stronger than Tat-SP4 by roughly 10-30 folds, respectively. The molecular modeling results suggest that, as shown in Fig. 4, the hydrocarbon staple on the newly obtained peptides is closer to the coiled coil domain surface instead of stretching into the solvent as in the case of Tat-SP4, which is a major factor accounting for their enhanced binding affinity. In summary, the new design strategy of adjusting the inserting position of hydrocarbon staple and adjusting the amino acid sequences of stapled peptides has yielded a collection of stapled peptides with promising activities at least in the in vitro binding assay.

In some embodiments, the Beclin 1-targeting stapled peptide provided by the present disclosure can effectively induce autophagy. The present embodiment focuses on i7-01s-20 and i7-01s-31, the two new stapled peptides having the strongest binding affinity to Beclin 1, for subsequent functional characterization. As shown in Fig. 7, trypan blue exclusion assay is first used to confirm that both i7-01s-20 and i7-01s-31 show negligible cytotoxicity on HEK293T cells at 10- and 20-µM concentrations respectively, after 24 h of treatment.

The impact of these two peptides on cellular autophagic activity is examined by detecting the protein levels of LC3 and p62, two molecular markers of autophagy. During the autophagy process, cytosolic protein LC3 is converted from its *apo* form (LC3-I) to lipidated form (LC3-II) for accumulation on autophagosomes. Thus, an increase in LC3-II level is regarded as a reliable indicator of elevated cellular autophagic activity. Similarly, p62 is an autophagy receptor that is degraded in the lysosome together with autophagic substrates. A decrease in p62 level is also commonly used as an indicator for higher autophagic activity, although some studies have shown that p62 is not as sensitive as LC3-II. The results show that treatment of HEK293T cells with i7-01s-31 at 10 and 20 µM for 3 hours induces noticeable albeit small increase in LC3-II levels (as shown in Fig.8a & 8b). Such increase becomes significantly stronger in the presence of the chloroquine (CQ), a lysosomal inhibitor (as shown in Fig. 8a & 8c). The protein level of p62 does not show any noticeable change after peptide treatments, either in the presence or absence of CQ (as shown in Fig. 8a, 8d & 8e). This pattern of change in LC3-II and p62 levels is comparable to what is observed for Tat-SP4 as reported in the previous study. Thus, the newly designed peptides i7-01s-20 and i7-01s-31, although showing 10-30 fold stronger binding affinity to Beclin 1 than Tat-SP4, actually induce autophagy with comparable efficacy.

In some embodiments, the Beclin 1-targeting stapled peptide provided by the present disclosure can effectively promote endolysosomal trafficking. As a scaffolding member of the PI3KC3 complex, Beclin 1 is essential for multiple PI3KC3-dependent processes including autophagy, endolysosomal trafficking and phagocytosis. To assess the efficacy of the newly designed peptides on the endolysosomal trafficking process, the ligand-induced endolysosomal degradation of cell surface receptors HER2 and EGFR is characterized by western blotting using SKBR3 breast cancer cell line that over-expresses both EGFR and HER2. In SKBR3 cells, treatment by the agonist EGF triggered slow degradation of EGFR and HER2, with about 50% remaining even after 24 hours (as shown in Fig. 9a, 9d & 9e). Tat-SP4 treatment at 2 µM does not show any noticeable impact on the degradation profile (as shown in Fig. 9b, 9d & 9e). In contrast, i7-01s-31 treatment at 2µM results in significant reduction of HER2 level, with about 50% decrease at 15 hours post EGF stimulation, and about 70% decrease at 24 hours (as shown in Fig. 9c & 9d). i7-01s-31 treatment also strongly promotes EGFR degradation, with approximately 60% reduction at 15 hours post EGF stimulation (as shown in Fig. 9c & 9e).

To further compare the potency of the newly designed peptides to Tat-SP4, the EGFR and HER2 degradation assays are repeated using higher concentrations of Tat-SP4. It is found that the concentration of Tat-SP4 should be elevated to 20- or 40-µM to achieve similar degradative effect on HER2 and EGFR levels (as shown in Fig. 10). The data indicate that the newly designed peptides with enhancing binding affinity to Beclin 1 also show significantly higher efficacy in terms of promoting endolysosomal trafficking of HER2 and EGFR.

In some embodiments, the Beclin 1-targeting stapled peptide provided by the present disclosure can potently inhibit the proliferation of HER2+ cancer cells. To evaluate the anti-proliferative potency of the newly designed peptides on EGFR- or HER2-driven cancer, trypan blue exclusion assay is used to measure the IC₅₀ values of the peptides on SKBR3 cells. The results show that while Tat-SP4 exerts a moderate anti-proliferative effect with IC₅₀ of 33.68 µM, both i7-01s-20 and i7-01s-31 are 2-5 times more potent with IC₅₀ values of 13.55 µM and 7.12 µM for i7-01s-31, respectively (as shown in Fig. 11a). Similarly, a five-day proliferation assay confirms that while Tat-SP4 reduced proliferation of SKBR3 cells by 30% at 20 µM (as shown in Fig. 11b), i7-01s-20 treatment at 20µM, or i7-01s-31 treatment at 10µM, show much higher dosage-dependent potency to completely abolish cell proliferation (as shown in Fig. 11c & 11d). These findings confirm that the newly designed stapled peptides inhibit proliferation of SKBR3 with significantly higher potency as compared to Tat-SP4.

In some embodiments, the Beclin 1-targeting stapled peptide provided in the present disclosure can induce necrotic cell death. Given the potent anti-proliferative efficacy of the newly designed peptides, the molecular mechanism of this process is investigated. Observation during the trypan blue exclusion assays confirm that SKBE3 cells undergo cell death, not cell growth arrest, after treatment by i7-01s-31. To investigate whether the peptide-induced cell death is associated with apoptosis, flow cytometry with annexin V and propidium iodide (PI) staining is performed in SKBR3 cells. Annexin V labelled with the FITC fluorophore specifically labels phosphatidylserine (PS), a marker for apoptosis because this membrane lipid is normally located on the inner leaflet of the plasma membrane but flips to the outer leaflet during apoptosis. Thus, an increase in Annexin V staining is a reliable and specific indicator of apoptosis. PI is a nucleic acid binding dye that labels cells after necrotic cell death because it can only reach the nucleus upon loss of plasma membrane integrity, a hallmark feature of necrosis. In contrast, the flow cytometry data indicate that treatment by doxorubicin, a chemotherapeutic agent known to induce apoptosis, led to significant increase of Annexin V positive cells, confirming that apoptosis is the major cause of cell death (as shown in Fig. 12). In contrast, treatment by Beclin 1-targeting peptides, either Tat-SP4 or the newly developed i7-01s-31, only leads to significant increase of PI positive staining cells but no increase of Annexin V positive cells (as shown in Fig. 12). Furthermore, the extent of necrotic cell death is dosage-dependent and correlates well with the potency of the peptide used, with i7-01s-31 inducing approximately 30% PI positive cells at 10 µM while Tat-SP4 only induced approximately 8% at same concentration (as shown in Fig. 12). Whether such necrotic cell death is caused by elevated autophagic activity or rapid degradation of EGFR and HER2 receptors need to be further investigated.

Designing peptides with a hydrocarbon staple to reinforce their α-helical structure has served as an effective strategy to generate molecular prototypes capable of specific binding to target proteins. The potency of a stapled peptide in modulating a specific biological activity is influenced by many factors. The most important consideration is to ensure that the stapled peptide, with the hydrocarbon staple, fits tightly and specifically to the intended binding site on the target protein. To achieve strong and specific binding, the amino acid sequence of the peptide is always subject to systematic scanning to identify residues that best fit onto the binding site of the target protein. For the hydrocarbon staple, the common approach is to place this moiety away from the intended binding site to avoid possible interference. Interestingly, the present disclosure discovers that a hydrocarbon staple placed at the edge of the binding interface can actually help strengthen the protein-peptide interaction by complementary hydrophobic interactions with the target protein. Here the presented data show that this strategy of placing the hydrocarbon staple close to the protein-peptide binding interface can help enhance the binding affinity and biological activity of Beclin 1-targeting stapled peptides. The Beclin 1 coiled coil domain forms a long α-helix with extended surface that lacks a well-defined binding site. The prototype peptides reported in the previous study are constructed as a short three-round amphipathic helix with 11 residues, thus affording only three hydrophobic residues (L192, L196 and V199) to interact directly with Beclin 1 coiled coil interface (as shown in Fig. 4a). In this study, "staple scanning" is conducted to explore all possible positions to install the hydrocarbon staple on the α-helical peptide. The molecular modeling results show that placing the staple close to the edge of the Beclin 1-peptide binding interface as in the i7-01s scaffold allows it to rest on the hydrophobic region on the molecular surface of Beclin 1 (as shown in Fig. 4b). These newly designed peptides showed 10-30 fold higher binding affinity to Beclin 1 *in vitro* and 2-5 fold stronger anti-proliferative potency in HER2 positive cancer cells *in vivo.* The present disclosure suggests that for stapled peptides targeting coiled coil domains that lack conventional binding sites, placing the hydrocarbon staple closer to the coiled coil surface may be an effective approach to enhance their binding affinity by effectively expanding the hydrophobic binding interface.

Although Beclin 1 is well regarded as a tumor suppressor, pharmacological targeting of Beclin 1 or the Beclin 1-mediated autophagy process for cancer therapy has been challenging. Extensive studies have delineated the functional role of autophagy in cancer as a double-edged sword, exerting both anti- and pro-tumor effect in context-dependent manner. Thus, simply enhancing or blocking autophagy may not yield sufficient clinical efficacy. Indeed, several clinical trials testing the anti-cancer efficacy of hydroxychloroquine, a commonly used autophagy inhibitor, show inconclusive results. Here an alternative approach is presented to modulate Beclin 1 activity in cancer cells using stapled peptides that bind to the Beclin 1 coiled coil domain with optimized specificity and potency. These peptides have the unique functional profile of up-regulating both autophagy and endolysosomal trafficking, a feature not seen in other autophagy inducers such as rapamycin or Tat-Beclin 1 peptide. In particular, with their binding affinity to Beclin 1 strengthened by 10-30 fold, these new peptides significantly enhance the endolysosomal degradation of cell surface oncogenic receptors EGFR and HER2, likely attenuating their downstream signaling as well. Given that EGFR or HER2-driven cancer cells particularly rely on EGFR and HER2 signaling pathways to sustain proliferation and metastasis, the cancer cells may be exceptionally sensitive to the Beclin 1-targeting peptides. In summary, the approach of targeting the Beclin 1 coiled coil domain with hydrocarbon-stapled peptides to enhance both autophagy and endolysosomal trafficking may be an effective therapeutic strategy for EGFR- or HER2-driven cancer.

In some embodiments, a method for designing a Beclin 1-targeting stapled peptide is also provided, the reagents and antibodies used are selected from the group consists of: Chloroquine (CQ), Epidermal Growth Factor (EGF), Protease inhibitor cocktail (Roche Diagnostics), Trypsin (Invitrogen), isopropyl-β-D- thiogalactopyranoside (IPTG), anti-β-actin antibody (Santa Cruz Biotechnology), anti-LC3 antibody (Abnova), anti-p62 antibody (Abnova), anti-EGFR antibody (Santa Cruz Biotechnology), anti-HER2 antibody (Cell Signaling Technology), Anti-Mouse IgG-HRP (Sigma-Aldrich), Anti-Rabbit IgG-HRP (Sigma-Aldrich); the computational design method includes:
Truncating an α-helical fragment (residues 191-205) on the Beclin 1 coiled coil domain as a template for designing stapled peptide binding to the Beclin 1 coiled coil domain. Molecular models of all stapled peptides are constructed using the MOE software (version 2019, released by the Chemical Computing Group). In the present disclosure, a linear hydrocarbon staple is added to connect two anchor residues that are separated by six residues (i.e., ith and *i*th+7 residues) on the template peptide sequence. A total of six pairs of anchor residues are considered (as shown in Fig. 2), namely i7-01s (connecting E195 and N202), i7-02s (connecting R191 and D198), i7-03s (connecting D198 and V205), i7-04s (connecting Q194 and K201), i7-05s (connecting E197 and K204), and i7-06s (connecting I193 and E200).

In the present disclosure, the i7-01s scaffold is chosen to perform mutations on the Native P1 sequence *in silico* in order to obtain new peptides with higher binding affinity. Except for the two anchor residues (i.e. E195 and N202), there are 13 residues on the Native-P1 sequence for possible mutation. Each of those 13 residues is mutated into the other 19 natural amino acid residues, which results in a total of 13*20 = 260 peptides. The complex model of each stapled peptide is derived directly from the model of i7-01s in complex with the Beclin 1 coiled coil domain. Those designed stapled peptide are subjected to molecular dynamics simulation and binding energy prediction (see the descriptions below for technical details). Based on the predicted binding energies, certain favorable single-point mutants are then manually selected and combined into new designs of multiple-point mutants to further enhance the interactions with the Beclin 1 coiled coil domain. This effort leads to a total of 75 stapled peptides with multiple mutations (fewer than five points) on the Native-P1 sequence. Among them, 17 stapled peptides are finally selected for chemical synthesis based on a balanced consideration of their predicted binding mode and binding affinity as well as their physicochemical properties (such as the total charge). Note that every designed stapled peptide is capped at the N- and C-terminal by an acetyl group and an amino group, respectively, in the molecular modeling.

Molecular dynamics (MD) simulations are employed to evaluate the interactions between the Beclin 1 coiled coil domain and the designed stapled peptides. All computations are conducted by using the CPU-version of the AMBER18 software or the GPU-version of the AMBER16 software. Beclin 1 as well as the stapled peptides are treated with the AMBER FF14SB force field. In particular, the hydrocarbon staple on each designed peptide is technically treated as two unnatural amino acid residues (i.e. R8G and S5G) connected by a double bond: R8G consists of seven methylene units stemming from the alpha-carbon on the peptide backbone in the R-configuration; whereas S5G consists of four methylene units stemming from another alpha-carbon on the peptide backbone in the S-configuration. The atomic charges assigned on the two unnatural residues are computed at the HF/6-31G* level with the Gaussian 09 software and then derived by using the restrained electrostatic potential (RESP) method in the AMBER software.

In the present disclosure, the crystal structure of the coiled coil domain of Beclin 1 (PDB entry 3Q8T) is used as the starting point for MD simulation. For the sake of convenience, the coiled coil domain is truncated to keep the segment between V213 and K264 only. The segment between R191 to V205 on the same crystal structure is truncated as the template for building the designed stapled peptides. The model of each stapled peptide is built by mutating certain residues into the desired ones. The resulting initial complex model is energetically minimized to release clashes between the stapled peptide the coiled coil domain. Then, the complex model is soaked in a cubic box filling with TIP3P water molecules. The whole system is prepared and energetically minimized through a multi-step process as described in the previous study. After minimization, the whole system is heated from 0 K to 300 K in 100 ps and then equilibrated for 500 ps under a constant pressure of 1 atm. Then, the resulting system is subjected to a MD simulation of 100 ns long. Three parallel simulations are conducted for each peptide to enhance conformational sampling. Technical settings of the above MD simulation process are also the same as in the previous work.

Binding energy of each stapled peptide to the Beclin 1 coiled coil domain is evaluated by using the Molecular Mechanics Generalized-Born Surface Area (MM-GB/SA) module in the AMBER software. The basic settings for applying MM-GB/SA are also the same as in the previous study. In the present disclosure, the binding energy of each stapled peptide is computed based on the last 20 ns section on the corresponding MD trajectory. Note that the configurational entropy term in the MM-GB/SA equation is not computed in the present disclosure because this part is assumed to be roughly the same for all peptides under consideration.

### Chemical synthesis of stapled peptide:

All peptide samples are purchased from GL Biochem (Shanghai) Ltd. Generally, the peptides are synthesized by automated solid-phase peptide synthesis, incorporating olefin-containing amino acids at the designated positions. Then, the hydrocarbon staple is formed on olefin-containing amino acids by ring-closing metathesis reaction using the Grubbs catalyst. Similar to the previous study, a cell-penetrating Tat sequence (YGRKKRRQRRR) is added to the N-terminal of each peptide to assist with intracellular delivery. Chemical structure and purity of the final products are characterized by HRMS and HPLC. HRMS and HPLC spectra of those peptides are provided as part of the Supporting Information. Stock solution of each obtained peptide is prepared by dissolving the sample in pure water to a concentration of 20 mM.

### Protein expression and purification:

The expression and purification of Beclin 1 coiled coil domain is performed as described previously. Briefly, the coiled coil domain of human Beclin 1 (residues 176-268) is cloned into the modified pET32a vector containing the human rhinovirus (HRV) 3C protease cleavage site and thioredoxin-6xHis fusion tag. Recombinant protein for the Beclin1 coiled oil domain is expressed in Escherichia coli BL21(DE3) and purified by Ni²⁺-NTA affinity chromatography (HisTrap HP, GE Healthcare) first and followed by size-exclusion chromatography (Superdex 75, GE Healthcare).

### Isothermal Titration Calorimetry (ITC):

ITC assays are carried out by MicroCal PEAQ-ITC Automated (Marvern) at 25 °C. All designed peptides and purified protein for the Beclin 1 coiled coil domain are dialyzed into the buffer containing 50 mM Tris, 150 mM NaCl at pH 7.4. For each titration assay, the injection syringe is loaded with 40 µl of peptide and the cell is loaded with Beclin 1 coiled coil domain protein. Typically, titrations consist of 19 injections, and each injection is performed at a time interval of 180 seconds to ensure that the titration peak returns to the baseline. The titration data are analyzed using the MicroCal PEAQ-ITC Analysis software and fitted by the one-site binding model.

### Cell culture:

HEK283T and SKBR3 cell lines are cultured in DMEM (Gibco, Grand Island, NY, USA) supplemented with 10% fetal bovine serum (Life Technologies), 1% penicillin G (100 U/ml), and streptomycin (100 mg/ml), and in a humidified incubator at 37 °C with 5% CO₂. All cell lines used in the experiments are mycoplasma detected negative by MycoAlertTM PLUS Mycoplasma Detection Kit (Lonza) before and during the experiment.

### Cell viability assay:

Cell viability is measured by trypan blue exclusion assay following the standard protocol provided by the manufacturer. SKBR3 cells are seeded on 96-well plates containing complete medium at a density of 1×10⁴ cells per well. Upon cell attachment, cells are treated with the indicated stapled peptide or vehicle control for 24 hours. Afterwards, the number of trypan blue positive staining cells and the number of total cells are manually counted under a light microscope (Olympus). Cell viability is calculated as the number of viable cells divided by the total number of cells. All experiments are repeated in triplicate and the mean is calculated.

### Immunoblot analysis:

Whole cell lysates are extracted using Laemmli sample buffer (62.5 mM Tris-HCl, pH 6.8, 2% SDS, 25% glycerol, 5% β-mercaptoethanol) supplemented with EDTA-free protease inhibitor cocktail (Roche). Protein concentration is determined using a standard Bradford assay before immunoblotting. Protein samples are separated by SDS-PAGE, transferred to a PVDF membrane (Millipore, USA), and incubated with the primary antibodies and HRP-conjugated secondary antibodies. Protein bands are visualized using ECL reagents. β-actin is used as the internal reference protein to control the loading amount of SDS-PAGE.

### EGFR/HER2 degradation assay:

HEK293T or SKBR3 cells in 6-well plate are washed with PBS two times and serum-starved overnight in DMEM medium. Endocytosis of EGFR and HER2 are induced by treatment with 200ng/mL of EGF at 37 °C. Cells are collected at the indicated time after EGF stimulation and lysed as described in immunoblot analysis.

### Flow Cytometry:

The degree of cellular apoptosis and necrosis is assessed by flow cytometry following the standard protocol provided by the manufacturer. Briefly, SKBR3 cells after the indicated peptide treatment are harvested by trypsin, labelled with Annexin V and PI (Invitrogen), subjected to flow cytometer system (BD Accuri C6), and analyzed by BD Accuri C6 software. The cell populations can be distinguished by the staining of Annexin V /PI. Typically, cellular population in the lower left quadrant is live cells (Annexin V⁻, PI⁻), the lower right quadrant represents early apoptotic cells (Annexin V⁺, PI⁻), the upper right quadrant represents late apoptotic cells (Annexin V⁺, PI⁺), the upper left quadrant represents necrotic cells (Annexin V⁻, PI⁺).

In summary, the present disclosure optimizes the Beclin 1-targeting stapled peptide by adjusting the installing position of the hydrocarbon staple and the amino acid sequence of the stapled peptide. The result shows that placing the hydrocarbon staple closer to the binding interface of the Beclin 1-stapled peptide enhances the binding affinity by 10-30 fold. Compared to other autophagy inducers like rapamycin and Tat-Beclin 1 peptide, the optimized stapled peptide of the present disclosure shows the unique profile of not only inducing autophagy but also significantly enhancing the endolysosomal degradation of cell surface oncogenic receptors EGFR and HER2 in HER2-positive cancer cells. Given the importance of EGFR and HER2 signaling pathways in cancer cell proliferation, the Beclin 1-targeting stapled peptide provided in the present disclosure may serve as effective therapeutic candidates for EGFR- or HER2-driven cancer.

It should be understood that the application of the present disclosure is not limited to the above-mentioned embodiments, and those skilled in the art can make transformations or modifications based on the above-mentioned descriptions, and all these transformations and modifications should belong to the protection of the appended claims of the present disclosure.

## Claims

1. A Beclin 1-targeting stapled peptide, wherein the stapled peptide comprises an amino acid sequence at least 66.7% identical to amino acid residues 191-205 of Beclin 1, a fifth amino acid residue and a twelfth amino acid residue in the stapled peptide are connected by a hydrocarbon staple to stabilize an α-helical structure of the stapled peptide, the amino acid sequence of the stapled peptide is SEQ ID NO.20 and SEQ ID NO.31.

2. The Beclin 1-targeting stapled peptide according to claim 1, wherein the stapled peptide contains one R-2-(7-octenyl) alanine residue and one (S)-2-(4-pentenyl) alanine residue.

3. A pharmaceutical composition, wherein comprising the stapled peptide according to any one of claims 1-2.
